(19) Europäisches Patentamt

European Patent Office

Office européen des brevets

(11) Publication number: **0 608 820 A1**

# (12) EUROPEAN PATENT APPLICATION

(21) Application number: **94101017.5**

(22) Date of filing: **25.01.94**

(51) Int. Cl.⁵: **G01N 33/52**, G01N 31/22, //C12Q1/26,C12Q1/28

(30) Priority: **27.01.93 JP 11482/93**

(43) Date of publication of application:
**03.08.94 Patentblatt 94/31**

(84) Designated Contracting States:
**CH DE FR GB IT LI**

(71) Applicant: **KYOTO DAIICHI KAGAKU CO., LTD.**
**57 Nishiaketa-cho**
**Higashikujo**
**Minami-ku**
**Kyoto-shi Kyoto-fu(JP)**

(72) Inventor: **Satoru, Mizutani, c/o Kyoto Daiichi**
**Kagaku Co.**
**Ltd, Nishiaketa-cho,**
**Higashikujo,**
**Minami-ku**
**Kyoto-shi, Kyoto-fu(JP)**
Inventor: **Syogo, Yamashita, c/o Kyoto Daiichi**
**Kagaku Co.**
**Ltd, Nishiaketa-cho,**
**Higashikujo,**
**Minami-ku**
**Kyoto-shi, Kyoto-fu(JP)**
Inventor: **Junji, Yoshioka, c/o Kyoto Daiichi**
**Kagaku Co.**
**Ltd, Nishiaketa-cho,**
**Higashikujo,**
**Minami-ku**
**Kyoto-shi, Kyoto-fu(JP)**

(74) Representative: **von Kreisler, Alek,**
**Dipl.-Chem.**
**Patentanwälte**
**von Kreisler-Selting-Werner**
**Bahnhofsvorplatz 1 (Deichmannhaus)**
**D-50667 Köln (DE)**

(54) **Analytical composition.**

(57) An analytical composition having at least one reagent layer and a protective layer which cover the reagent layer and is substantially free from any reagent that participates a reaction for measurement, which can prevent mutual contamination of test pieces of the compositions caused by vibration in the production or transportation step, and also contamination of a test piece or a test sample, whereby accurate data are obtained and diagnosis of the disease is made accurately.

Rank Xerox (UK) Business Services
(3.10/3.09/3.3.4)

## BACKGROUND OF THE INVENTION

### Field of the Invention

The present invention relates to a dry type analytical composition which can be used, without beforehand preparation of an agent, to detect or analyze a substance in a test sample easily, accurately and quickly, so as to immediately know a cause or condition of a disease of a patient in a clinical examination field.

In a general examination of urine or a biochemical examination of serum, in general, a set of several test items are examined to grasp the cause or condition of the disease comprehensively. The present invention relates to an analytical composition which can provide more accurate data in the multi-item analysis by preventing mutual contamination of agents between the test items.

### Description of the Related Art

To examine urine in the clinical examination field, a test strip is used, which is prepared by impregnating a solution containing various agents in a filter paper sheet, drying the filter paper sheet cutting the filter paper sheet in a suitable size, for example, 5 mm x 5 mm, and adhering the cut piece on a plastic base film. In the urine examination, the test strip is dipped in the urine sample and removed, and after a certain time, a degree of color development of the agent is measured by naked eyes or with an analyzing equipment.

In the biochemical examination of the serum, a test strip is used, which is prepared by coating a reagent layer or reagent layers on a plastic film and laminating or mounting a porous spreading layer over the reagent layer(s).

In the examination, a certain amount of the serum is dropped on the spreading layer and after a certain time, an amount of a dye, which is generated in the reagent layer by a reaction of the reagent with a substance to be analyzed, is measured using an equipment such as a spectrophotometer.

In the case of the test strip for urine examination, since three to nine items are analyzed at the same time, the number of test pieces corresponding to the number of test items are adhered to the plastic base film, and a number of test strips for multi-item examination are stored in a glass bottle or an aluminum can.

Usually, a filter paper sheet is used as a base matrix to carry the reagent. Filter paper fibers drop off from the test strips during production or transportation of the test strips and adhere to a surface of other test pieces, whereby the other test pieces are color developed in a spot form and an error may be caused in the analysis.

In addition, since the test strip is dipped in the sample urine, the reagent is dissolved in the sample urine and tends to contaminate the sample urine. The contaminated sample urine cannot be used in the further analysis.

To solve the above described problems, many analytical compositions have been proposed.

Japanese Patent Publication No. 39189/1973 (corres. to Canadian Patent No. 964,175) discloses a method for producing a storable test paper which comprises at least one test piece sealed by a pair of plastic films, wherein the upper and lower surfaces of the test piece are completely covered, while at least one edge of the test piece is not sealed by the plastic films. The disclosed pair of the plastic films are made of water-insoluble plastic (e.g. polyvinyl chloride, polyterephthalate, polyethylene-laminated polyterephthalate, polypropylene, super polyamide, etc.) and fusion bonded by thermal impulse. While the test piece covered by the water-insoluble plastic films has improved storage stability, a test sample cannot permeate the plastic films. Then, the test sample reaches the test piece through an opening on the unsealed edge.

Japanese Patent KOKAI Publication No. 38861/1990 discloses an examination article comprising a reagent layer and a colorless transparent covering member which is mounted to cover the reagent layer. Since the covering member is made of a water - resistant resin such as polyethylene, polypropylene, polycarbonate, polyester and the like, an opening through which the test sample reaches the test piece should be formed in the covering member. In the production method disclosed in this KOKAI Publication, the covering member is fixed to a support member with an adhesive or by thermal impulse to cover the reagent layer.

In the biochemical examination of the serum, plural items are analyzed with the single sample serum, and the cause and condition of disease are grasped according to comprehensive data. In such case, a necessary amount of serum is collected and dropped on each test piece for the respective test item.

A tip end of a nozzle may be slightly contact to the surface of test piece on dropping the serum, and the reagent in the test piece may adhere to the tip end of the nozzle.

When a drop of the serum is provided on the subsequent test piece, the agent from the previous test piece is mixed in the subsequent test piece to cause an error in measurement.

Further, when another sample serum is collected with the contaminated nozzle, the serum is contaminated with the reagent so that it cannot be analyzed.

Each test piece of the dry type test strip contains a respective reagent in a high concentration to measure an amount of a substance or enzyme contained in a body fluid such as urine, serum, cerebrospinal liquid, and so on.

The test strip is cut or blanked in a stick or mount form to make its handling easy in the measurement.

For further convenience, plural test pieces are adhered to one strip.

In the cutting or blanking step, a physical stress is applied on the test pieces, so that a part of the test piece is peeled to form fragments or powder. When the fragments or powder adhere to the adjacent reagent layer, it is colored in spots or discolored to give an erroneous measured value.

For example, glucose oxidase which is used to measure glucose in the serum and a color developing system therefore are deposited on a reagent layer for measuring uric acid, a total amount of uric acid and glucose is measured. Since an amount of glucose is contained in an amount ten times or more that of uric acid in the serum, the above contamination causes a great error in the measurement of uric acid.

When the tip end of the nozzle for providing the sample liquid contacts the reagent layer in the course of measurement, the reagent is adhered to the tip end of the nozzle, which results in the error in the measurement.

In addition, when the sample is collected using the nozzle carrying the reagent on its tip end, the carried reagent is included in the test sample and the reaction of the reagent with substances in the sample gradually proceeds, so that properties of the test sample are changed from the original properties of the sample.

The only way to avoid the mutual contamination of the reagents is to control the production steps carefully to prevent the formation of fragments or powder of the reagent layers.

However, it is almost impossible to remove undetectable fine fragments or powder.

## SUMMARY OF THE INVENTION

An object of the present invention is to provide an analytical composition or strip which can prevent mutual contamination of reagents contained in respective test pieces during storage or transportation.

According to the present invention, there is provided an analytical composition comprising at least one reagent layer and a protective layer which covers said at least one reagent layer and is substantially free from any reagent that participates a reaction for measurement.

## DETAILED DESCRIPTION OF THE INVENTION

The analytical composition or strip of the present invention is used for measuring a substance or substances contained in a body fluid such as serum, urine, cerebrospinal liquid and so on. Typically, it is an analytical composition used in the examination of urine or biochemical examination of serum.

As the reagent, any of conventionally used ones is used in the present invention. For examine, a combination of glucose oxidase and peroxidase is used for the measurement of glucose, and a combination of uricase and peroxidase is used for the measurement of uric acid.

A protective layer used in the present invention is made of a material which is not peeled off by physical stress but can be dissolved in the test sample liquid in a very short time.

As a material which satisfies such requirements, any one having an ability of forming a hydrophilic film can be used. Examples of such material are gelatin, gelatin derivatives, starch, starch derivatives, alginate salts, carboxymethylcellulose, hydroxymethylcellulose, methylcellulose, polyvinyl alcohol, polyvinyl pyrrolidone, polyacrylamide, polyacrylic acid salt, gantrez, and the like, or mixtures thereof.

If necessary, a wetting agent, a plasticizer, a surfactant, a pH regulator, and so on may be added to the protective layer as a film modifier.

Further, the analytical composition will have better properties when the protective layer contains a compound which will decompose a substance interfering the reaction for measurement (e.g. ascorbic acid, bilirubin, etc.). Examples of such compound are ascorbic acid oxidase, bilirubin oxidase, and the like.

The protective layer can be formed by applying a solution of the film forming material on the reagent layer or the test strip by, for example, gravure or silk printing or spraying, and drying the applied solution.

A thickness of the dried protective layer is preferably from 1 to 50 $\mu$m, more preferably from 5 to 30 $\mu$m.

PREFERRED EMBODIMENTS OF THE INVENTION

The present invention will be explained further in detail by the following Examples, which will not limit the scope of the present invention in any way.

Comparative Example 1 (without protective layer)

Composition of a glucose-measuring reagent layer:

| | |
|---|---|
| Glucose oxidase | 40 KU |
| Peroxidase | 100 KU |
| 4-Aminoantipyrine | 1 g |
| 3,5-Dimethoxy-N-ethyl-N-(2-hydroxy-3-sulfopropyl)aniline.sodium | 2 g |
| 0.2 M phosphate buffer (pH 7.0) | 100 ml |
| Sodium alginate | 1 g |
| 20 % Triton X-100 | 1 ml |

The above reagent composition was coated on an opaque polyethylene terephthalate film. Then, a porous polyester fabric was laminated on the coated reagent layer and dried at 50°C for 30 minutes. A dry thickness of the reagent layer was 20 $\mu$m.

Example 1 (with protective layer)

Composition of protective layer:

| | |
|---|---|
| Sodium alginate | 0.5 g |
| 0.05 M phosphate buffer (pH 7.0) | 100 ml |
| 20 % Triton X-100 | 0.2 g |

On the reagent layer formed in the same manner as in Comparative Example 1, the above protective layer composition was continuously coated with a gravure coating roll and dried at 50°C for 30 minutes. A dry thickness of the protective layer was 10 $\mu$m.

Comparative Example 2 (without protective layer)

Composition of uric acid-measuring reagent layer:

| | |
|---|---|
| Uricase | 5 KU |
| Peroxidase | 20 KU |
| 4-Aminoantipyrine | 100 mg |
| 3,5-Dimethoxy-N-ethyl-N-(2-hydroxy-3-sulfopropyl)aniline.sodium | 500 mg |
| 0.5 M phosphate buffer (pH 7.0) | 100 ml |
| Hydroxypropylcellulose | 3 g |
| 20 % Triton X-100 | 1 ml |

The above reagent composition was coated on an opaque polyethylene terephthalate film. Then, a porous polyester fabric was laminated on the coated reagent layer and dried at 50°C for 30 minutes. A dry thickness of the reagent layer was 15 $\mu$m.

4

Example 2 (with protective layer)

Composition of protective layer:

| | |
|---|---|
| Poly(sodium acrylate) | 2 g |
| 0.05 M phosphate buffer (pH 7.0) | 100 ml |
| 20 % Triton X-100 | 0.2 g |

On the reagent layer formed in the same manner as in Comparative Example 2, the above protective layer composition was continuously coated with a gravure coating roll and dried at 50°C for 30 minutes. A dry thickness of the protective layer was 20 μm.

Each of the dry type test sheets produced in Comparative Examples 1 and 2 and Examples 1 and 2 was cut to a size of 5 mm x 5 mm and adhered to one end of a plastic film strip (5 mm x 100 mm) with a double sided adhesive tape.

Experiment 1

Each 50 test strips produced in Comparative Example 1 (for glucose without protective layer) and Comparative Example 2 (for uric acid without protective layer) were stored in an aluminum bottle and sealed. Then, the bottle was shaken at 300 rpm for one hour.

In the same way, each 50 test strips produced in Example 1 (for glucose with protective layer) and comparative Example 2 (for uric acid without protective layer) were stored in the aluminum bottle and shaken.

On one of 100 test strips of Comparative Example 2 which were shaken themselves and one of the 50 test strips of Comparative Example 2 which were shaken together with the 50 strips of Example 1 or Comparative Example 1, 10 μl of human serum was dropped and incubated at 37°C for 5 minutes. Then, a reflectance was measured on the uric acid measuring reagent piece.

From the measured reflectance (%), a K/S value was calculated according to the following Kubelka-Munk equation:

$$K/S = (1-R)^2/2R$$

wherein R is a reflectance/100.

The results are shown in Table 1.

Table 1

| Run No. | Uric acid value (K/S value) of test strips of Com. Ex. 2 | | | Uric acid value (K/S value) of test strips of Example 2 |
|---|---|---|---|---|
| | Shaken with test strips of | | | Shaken with test strips of |
| | Com. Ex. 2 (Reference) | Com. Ex. 1 (no protective layer) | Exam. 1 (protective layer) | Example 1 (protective layer) |
| 1 | 1.01 | 1.15 | 1.02 | 1.03 |
| 2 | 1.02 | 1.09 | 1.03 | 1.06 |
| 3 | 1.06 | 1.08 | 1.00 | 1.02 |
| 4 | 1.01 | 1.13 | 1.05 | 1.03 |
| 5 | 1.02 | 1.20 | 1.07 | 1.01 |
| 6 | 1.02 | 1.18 | 1.02 | 1.03 |
| 7 | 1.04 | 1.10 | 1.00 | 1.00 |
| 8 | 1.01 | 1.05 | 1.03 | 1.03 |
| 9 | 1.00 | 1.17 | 1.01 | 1.05 |
| 10 | 1.02 | 1.10 | 1.02 | 1.03 |
| Av. | 1.021 | 1.125 | 1.025 | 1.029 |
| C.V. | 1.7 | 4.3 | 2.1 | 1.7 |

When the test strips of Comparative Example 2 were shaken by themselves, the average value of uric acid concentrations and the reproducibility, namely the C.V. were good. As the C.V. is smaller, the deviation is small.

When the test strips of Comparative Example 2 were shaken together with the test strips of Comparative Example 1 having no protective layer, the measured values of uric acid concentration were apparently higher than those obtained in the reference experiment, and the deviation was larger.

When the test strips of Comparative Example 2 were shaken together with the test strips of Example 1, the measured values and reproducibility were substantially the same as those obtained in the reference experiment.

When both the reagent layers for measuring glucose and uric acid had the protective layers, the measured values and reproducibility were closest to those obtained in the reference experiment.

Experiment 2

Ten microliters of human serum containing 115 mg/dl of glucose and 7 mg/dl of uric acid were collected with an automated pipette and dropped on the glucose-measuring piece of the test strip and then dropped on the uric acid-measuring piece of the test strip. After incubating the strips at 37°C for 5 minutes, the reflectance on the test piece was measured using light having the wavelength of 590 nm.

The above experiments compared a degree of contamination of the uric acid-measuring test piece with the pipette nozzle which had been contacted to the glucose-measuring test piece.

The results are shown in Table 2.

Table 2

| Run No. | Measured value of uric acid concentration (K/S value) | | |
|---------|-----------------------------------|----------------------------------------------------------|----------------------------------------------------------|
| | Example 2 (reference) | Comp. Ex 1 (for glucose) (no protective layer) ⇓ Comp. Ex. 2 (for uric acid) (no protective layer) | Example 1 (for glucose) (with protective layer) ⇓ Example 2 (for uric acid) (with protective layer) |
| 1 | 1.05 | 1.35 | 1.03 |
| 2 | 1.07 | 1.23 | 1.03 |
| 3 | 1.01 | 1.40 | 1.06 |
| 4 | 1.06 | 1.35 | 1.00 |
| 5 | 1.03 | 1.36 | 1.04 |
| 6 | 1.04 | 1.30 | 1.05 |
| 7 | 1.04 | 1.42 | 1.02 |
| 8 | 1.00 | 1.26 | 1.02 |
| 9 | 1.02 | 1.29 | 1.05 |
| 10 | 1.03 | 1.37 | 1.07 |
| Av. | 1.035 | 1.333 | 1.037 |
| C.V. | 2.1 | 4.6 | 2.0 |

When the uric acid alone was measured as in the reference experiment, no material problem arose. However, when the glucose concentration was first measured and then the uric acid concentration was measured using the same pipette, the measured values of uric acid concentrations and the deviation were apparently increased in the absence of protective layer, since the glucose - measuring reagent was adhered to the tip end of the nozzle in the glucose measuring step so that the total concentration of glucose and uric acid was measured in the uric acid measuring step to create a positive error.

But, when the protective layer was provided on the glucose-measuring reagent layer, neither the measured value of uric acid concentration nor the deviation was increased.

As seen from the above experiments, when the analytical composition or strip of the present invention is used, the mutual contamination of the test pieces of the strips caused by vibration in the production or

7

transportation step can be prevented.

In addition, the contamination of the test piece or the test sample can be avoided when the test sample is dropped on the test piece, whereby accurate data are obtained and diagnosis of the disease is made accurately.

## Claims

1. An analytical composition comprising at least one reagent layer and a protective layer which covers said at least one reagent layer and is substantially free from any reagent that participates a reaction for measurement.

2. The analytical composition according to claim 1, which is of a strip form.

3. The analytical composition according to claim 1, wherein said protective layer is made of a hydrophilic polymer.

4. The analytical composition according to claim 3, wherein said hydrophilic polymer is at least one polymer selected from the group consisting of gelatin, gelatin derivatives, starch, starch derivatives, alginate salts, carboxymethylcellulose, hydroxymethylcellulose, methylcellulose, polyvinyl alcohol, polyvinyl pyrrolidone, polyacrylamide and polyacrylic acid salt.

5. The analytical composition according to claim 1, wherein said protective layer has a thickness of 1 to 50 $\mu$m.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | WO-A-88 09824 (NATIONAL DIAGNOSTIC PRODUCTS (AUSTRALIA) PTY. LIMITED) | 1-5 | G01N33/52 G01N31/22 //C12Q1/26, C12Q1/28 |
| Y | * page 4, line 7 - page 5, line 29; claims * | 4 | |
| X | WO-A-91 01490 (PB DIAGNOSTIC SYSTEMS, INC.) * page 4, line 11 - page 5, line 4 * | 1-5 | |
| X | DATABASE WPI Week 8812, Derwent Publications Ltd., London, GB; AN 88-078255 & DD-A-251 002 (AKAD WISSENSCHAFT DDR) 28 October 1987 * abstract * | 1-5 | |
| X | DATABASE WPI Week 8647, Derwent Publications Ltd., London, GB; AN 86-305778 & DD-A-237 676 (AKAD WISSENCHAFT DDR) 23 July 1986 * abstract * | 1-5 | TECHNICAL FIELDS SEARCHED (Int.Cl.5) G01N |
| X | WO-A-90 10868 (BADISCHE TABAKMANUFACTEUR ROTH-HÄNDLE GMBH) * page 16, line 33- page 17, line 7; page 19, lines 1-14. * | 1-4 | |
| X | WO-A-90 05914 (PB DIAGNOSTIC SYSTEMS, INC.) * page 8, line 28 - page 9, line 30; figures * | 1-3 | |
| Y | WO-A-92 12239 (BOEHRINGER MANNHEIM CORPORATION) * page 7, line 13 - page 7, line 20 * | 4 | |

-/--

The present search report has been drawn up for all claims

| Place of search | Date of completion of the search | Examiner |
|---|---|---|
| THE HAGUE | 25 April 1994 | Hitchen, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Application Number

EP 94 10 1017

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A | EP-A-0 264 079 (FUJI FILM CO., LTD.) <br> * page 5, line 24 - page 7, line 9 * <br> ----- | 1-4 | |

**TECHNICAL FIELDS SEARCHED (Int.Cl.5)**

The present search report has been drawn up for all claims

| Place of search | Date of completion of the search | Examiner |
|---|---|---|
| THE HAGUE | 25 April 1994 | Hitchen, C |